# EUROPEAN PATENT APPLICATION

(11) **EP 1 679 516 A1**
(43) Date of publication of application: **12.07.2006**
(21) Application number: 04773491.8
(22) Date of filing: 22.09.2004
(51) Int. Cl.: G01N 33/543

(54) **METHOD OF IMMUNOREACTION MEASUREMENT AND, FOR USE THEREIN, REAGENT, KIT AND OPTICAL CELL**

(30) Priority: 26.09.2003 JP 2003336199
(71) Applicant: MATSUSHITA ELECTRIC INDUSTRIAL CO., LTD., Kadoma-shi, Osaka 571-8501 (JP)
(72) Inventor: KITAWAKI, Fumihisa, Matsushita El. Ind. Co., Ltd., Chuo-ku, Osaka-shi, Osaka 540-6319 (JP); KAMEI, Akihito, Matsushita El. Ind. Co., Ltd., Chuo-ku, Osaka-shi, Osaka 540-6319 (JP); KAWAMURA, Tatsurou, Matsushita El. Ind. Co., Ltd., Chuo-ku, Osaka-shi, Osaka 540-6319 (JP)
(74) Representative: Ehnis, Tobias
(86) International application number: PCT/JP2004/014317
(87) International publication number: WO 2005/031353

(57) **Abstract**

In the present invention, there is provided a method for measuring, under an acidic pH condition, a subject substance in a specimen. The method includes: step A for forming a reaction system by mixing the specimen and an antibody against the subject substance in the specimen; and step B for measuring an antigen-antibody reaction in the reaction system, wherein a pH of the reaction system is set to be acidic, and the relationship between a pI of the antibody and the pH of the reaction system is set to be pI > pH. According to the present invention, a measurement accuracy can be enhanced in an immune measurement reaction system basically using an acidic buffer solution, particularly in a region of low concentration for an antigen.

## Description

### TECHNICAL FIELD

The present invention relates to an immunoreaction measurement method allowing a measurement of a subject substance contained in a specimen under an acidic pH condition, and to a reagent, a kit, and an optical cell for use therein.

### BACKGROUND ART

In order to diagnose various diseases, and examine progression of symptoms, examination for a protein that is characteristic to the diseases and that is present in the human body fluid has been commonly practiced in the field of medicine and clinical examination. For example, if an individual is infected with hemolytic streptococcus, an antibody named ASO is generated in the blood for resistance. Therefore, by testing an amount of the antibody, as a measurement item, in the blood, whether the individual is infected with hemolytic streptococcus can be examined. Also, it is known that an RF (a rheumatoid factor) frequently appears in the serum of RA (rheumatoid arthritis) patients. In the serum of the RA patients, there is a substantial galactose deficiency in the carbohydrate IgG compared to a carbohydrate IgG in healthy people, thereby resulting in an abnormal glycosylation. It is considered that the galactose deficient IgG is generated at an early stage of an RA onset and an autoantibody against the garactose deficient IgG takes part in the onset of arthritis or the like. Hence, through testing for, as a measurement item, the antibody against the galactose deficient IgG in the serum by using a galactose deficient IgG antigen, a diagnosis for RA is possible.

In a measurement for the content of such proteins, mainly, highly-specific immunoreaction measurement methods have been widely used. Among them, nephelometry (or turbidimetry) is a method for detecting an agglutination complex generated in a specific antigen-antibody reaction, and is basically performed in a homogenous solution thereby being a quantitatively favorable method. Also, the method allows an easy operation since the measurement can be performed without separating the antigen-antibody complex from non-reacting antibodies and antigens.

On the other hand, in the measurement using nephelometry, a zone phenomenon commonly occurs in an antigen excess region. Therefore, depending on a measurement item, there has been a problem that an accurate measurement cannot be performed in a concentration region in which a measurement is required. The "zone phenomenon" refers to a phenomenon that, in a case where the amount of one of an antigen and an antibody exceeds the equivalent weight region thereof in which the antigen and the antibody form the largest amount of agglutination complexes, generation of the agglutination complex is hindered, and therefore, a measurement value (for example, scattered light intensity) becomes smaller than a proper value.

In an actual homogeneous immunoreaction measurement, an antibody is often used to measure a concentration of an antigen which is a subject substance. Generally, in an antigen concentration region where the zone phenomenon has not occurred, a huge molecular chain (an agglutination complex) composed of complexes in which antibodies and antigens are alternately linked together is generated. When it is assumed that the antibody concentration is constant, the amount or the size of the molecular chain is increased depending on the antigen concentration, and therefore, by measuring variations in the amount or the size of the molecular chain through variations in an optical amount (for example, variations in a scattered light intensity or in a transmitted light intensity), the antigen concentration can be quantitatively determined. However, in an antigen excess region, because the amount of the antigen exceeds that of the antibody, antigen binding sites of the antibodies are saturated, causing a difficulty in forming a bridge structure via the antigen. Therefore, generation of the above-described molecular chain is hindered, thereby causing an inability of determining the variation in the amount or the size of the antigen-antibody complex through the variation in the optical amount, resulting in a small measurement value. Consequently, the result is difficult to be distinguished from that in a case where the antigen concentration is low. Accordingly, when the zone phenomenon occurs, as well as the problem that a concentration of a subject substance cannot be accurately measured, a problem of false-negative emerges.

As a reaction system for relaxing such a zone phenomenon occurred in the antigen excess region, a method using an acidic buffer containing polyvalent carboxylic acid or polyvalent carboxylate salt has been disclosed (for example, Japanese Laid-Open Patent Publication No. 2003-66047). Generally, the measurement using the antigen-antibody reaction is often performed on a neutral to slightly alkaline side. However, in Japanese Laid-Open Patent Publication No. 2003-66047, the reaction takes place under a slightly acidic condition in order to suppress the zone phenomenon, thereby enabling a more accurate quantitative measurement especially in a broader range for the antigen excess region.

### DISCLOSURE OF THE INVENTION

As described above, the immunoreation measurement method using the acidic buffer is excellent in solving the problem caused by the zone phenomenon in the antigen excess region. However, on the other hand, when an antigen concentration is zero, there is a tendency that a measurement value (blank value) becomes increased.

The present invention is obtained in view of such a situation. In other words, the object of the present invention is to provide an immunoreaction measurement method for measuring a subject substance under an acidic pH condition based on an antigen-antibody reaction, and a regent, a kit, and an optical cell for use therein which allow a highly accurate quantitative measurement even in a case where an antigen concentration is low.

As a result of later described studies, the present inventors found that, by selecting a pI of an antibody molecule and a pH of a reaction system to be (pI value of the antibody) > (pH value of the reaction system), a non-specific agglutination of the antibody molecules is reduced, thereby preventing a blank value from becoming increased. The reason for the above is considered that, by setting the pI of the antibody and the pH of a solution composing the reaction system as above, an individual antibody molecule is positively charged in the solution, and the positively charged antibody molecules electrically repulse with each other, thereby suppressing the non-specific agglutination of the antibody molecules. Accordingly, especially in the case where the antigen concentration is low, in an event of measuring the reaction amount for the antigen-antibody reaction, measuring of the reaction amount for the non-specific agglutination between the antibodymolecules, together with the reaction amount for the antigen-antibody reaction, can be prevented, whereby an improvement for a S/N ratio can be expected, and an accuracy in the measurement is enhanced.

The present invention provides a measurement method for measuring a subject substance in a specimen under an acidic pH condition, and the method includes step A of forming a reaction system by mixing the specimen and an antibody against the subject substance in the specimen, and step B of measuring an antigen-antibody reaction in the reaction system in which a pH thereof is set to be acidic and the relationship between a pI of the antibody and the pH of the reaction system is set to be pI > pH.

In a preferred embodiment of the measurement method of the present invention, the amount of an antigen-antibody complex formed between the subject substance and the antibody is measured in the step B.

In a preferred embodiment of the measurement method of the present invention, the difference between the pI and the pH is equal to or more than about 0.5. More preferably, the difference between the pI and the pH is equal to or more than about 1.0. Still more preferably, the difference between the pI and the pH is equal to or more than about 1.5.

In a preferred embodiment of the measurement method of the present invention, the pH of the reaction system is in a range of about 4 to 6. Most preferably, the pH of the reaction system is in a range of about 4.5 to 5.0.

In a preferred embodiment of the measurement method of the present invention, in the step A, the reaction system is formed by mixing the specimen, the antibody, and a buffer.

In a preferred embodiment of the measurement method of the present invention, the reaction system includes an organic acid or an organic acid salt. More preferably, the organic acid or the organic acid salt is polyvalent carboxylic acid or polyvalent carboxylate salt. Still more preferably, the polyvalent carboxylic acid or the polyvalent carboxylate salt is either: tricarboxylic acid or tricarboxylate salt; or dicarboxylic acid or dicarboxylate salt.

In a preferred embodiment of the measurement method of the present invention, the antibody is a monoclonal antibody, a polyclonal antibody, or a labeled antibody.

In a preferred embodiment of the measurement method of the present invention, the step B of measuring includes a measurement of an optical parameter attributed to the amount or the size of the complex.

In another aspect, the present invention provides a reagent for measuring a subject substance in a specimen under an acidic pH condition based on an antigen-antibody reaction principle, and the reagent contains an antibody against the subject substance, and is prepared such that a pI of the antibody with respect to the acidic pH is pI > pH.

In a preferred embodiment of the reagent of the present invention, the difference between the pI and the pH is equal to or more than about 0.5. More preferably, the difference between the pI and the pH is equal to or more than about 1.0. Still more preferably, the difference between the pI and the pH is equal to or more than about 1.5.

In a preferred embodiment of the reagent of the present invention, the pH is in a range of about 4 to 6. Most preferably, the pH is in a range of about 4.5 to 5.0.

In a preferred embodiment of the reagent of the present invention, the reagent contains an organic acid or an organic acid salt. More preferably, the organic acid or the organic acid salt is polyvalent carboxylic acid or polyvalent carboxylate salt. Still more preferably, the polyvalent carboxylic acid or the polyvalent carboxylate salt is either: tricarboxylic acid or tricarboxylate salt; or dicarboxylic acid or dicarboxylate salt.

In a preferred embodiment of the reagent of the present invention, the antibody contained in the reagent is a monoclonal antibody, a polyclonal antibody, or a labeled antibody.

In a preferred embodiment of the reagent of the present invention, the reagent is provided in a dry state. For example, the reagent can be freeze-dried, and is provided. In another preferred embodiment, the reagent is provided as a solution.

In still another aspect, the present invention provides a measurement kit for measuring a subject substance in a specimen under an acidic pH condition based on an antigen-antibody reaction principle, and the kit includes a buffer solution and a reagent containing an antibody against the subject substance, and the reagent is prepared such that a pI of the antibody with respect to the acidic pH is pI > pH.

In a preferred embodiment of the kit of the present invention, the difference between the pI and the pH is equal to or more than about 0.5. More preferably, the difference between the pI and the pH is equal to or more than about 1.0. Still more preferably, the difference between the pI and the pH is equal to or more than about 1.5.

In a preferred embodiment of the kit of the present invention, a pH of the buffer solution is in a range of about 4 to 6. Most preferably, the pH of the buffer solution is in a range of about 4.5 to 5.0.

In a preferred embodiment of the kit of the present invention, the buffer solution contains an organic acid or an organic acid salt. More preferably, the organic acid or the organic acid salt is polyvalent carboxylic acid or polyvalent carboxylate salt. Still more preferably, the polyvalent carboxylic acid or the polyvalent carboxylate salt is either: tricarboxylic acid or tricarboxylate salt; or dicarboxylic acid or dicarboxylate salt.

In a preferred embodiment of the kit of the present invention, the antibody is a monoclonal antibody, a polyclonal antibody, or a labeled antibody.

In a preferred embodiment of the kit of the present invention, the antibody is a mixture of at least equal to or more than two types of monoclonal antibodies each recognizing a different epitope.

In a preferred embodiment of the kit of the present invention, the reagent is provided in a dry state. For example, the reagent can be freeze-dried, and is provided.

In a preferred embodiment of the kit of the present invention, the buffer solution and the reagent are mixed together immediately before the use for measuring the subject substance, and used as a test solution.

In still another aspect, the present invention provides an optical cell for measuring a subject substance in a liquid specimen under an acidic pH condition based on an antigen-antibody reaction principle. The optical cell has an aperture portion used for accepting the liquid specimen, an antibody against the subject substance is held in the cell so as to be dissolvable in the liquid specimen, a pH of the reaction system formed by mixing the liquid specimen and the antibody against the subject substance is acidic in the cell, and the relationship between the pH of the reaction system and the pI of the antibody in the cell is pI > pH. Here, a buffer may be held in the optical cell.

In a preferred embodiment of the optical cell of the present invention, the optical cell further has a connection aperture portion for connecting with a plunger so as to be able to take in the liquid specimen from the aperture portion by using the plunger.

In a preferred embodiment of the optical cell of the present invention, the optical cell is constructed in a material having substantially flat surfaces and being optically transparent in a visible light region. As such a material, a silica glass, a polystyrene, a polypropylene, or the like can be given. Especially, the polystyrene is preferred.

In a preferred embodiment of the optical cell of the present invention, the difference between the pI of the antibody and the pH of a buffer solution is equal to or more than about 0.5, and more preferably, is equal to or more than about 1.0. Still more preferably, the difference between the pI and the pH is equal to or more than about 1.5.

In a preferred embodiment of the optical cell of the present invention, the pH of the buffer solution is in a range of about 4 to 6. Most preferably, the pH of the buffer solution is in a range of about 4.5 to 5.0.

In a preferred embodiment of the optical cell of the present invention, the buffer contained in the optical cell of the present invention is an organic acid or an organic acid salt. Preferably, the organic acid or the organic acid salt is polyvalent carboxylic acid or polyvalent carboxylate salt. Still more preferably, the polyvalent carboxylic acid or the polyvalent carboxylate salt is either: tricarboxylic acid or tricarboxylate salt; or dicarboxylic acid or dicarboxylate salt.

In a preferred embodiment of the optical cell of the present invention, the antibody used in the optical cell of the present invention is a monoclonal antibody, a polyclonal antibody, or a labeled antibody. Still more preferably, the antibody is a mixture of at least equal to or more than two types of monoclonal antibodies each recognizing a different epitope.

In a preferred embodiment of the optical cell of the present invention, an antibody reagent contained in the optical cell of the present invention is provided in a dry state. For example, the antibody reagent can be freeze-dried, and is provided.

In a preferred embodiment of the optical cell of the present invention, the buffer is held in a dry state.

According to the present invention, when a measurement is performed for the amount of a subject substance in a specimen under an acidic pH condition based on an antigen-antibody reaction principle, a formation of a non-specific agglutination between antibody molecules can be suppressed. Accordingly, the amount of the antigen-antibody reaction can be accurately detected even in a case where an antigen concentration is low, thereby allowing a highly precise or highly sensitive measurement. Further, by removing a non-specific reaction, even in the case where the antigen concentration is low, a quantitative measurement based on the antigen-antibody reaction can be performed with high reproducibility.

Further, it is also possible to optimally eliminate a time effect, on a measurement value, which arises from the non-specific agglutination endlessly agglutinating over time, for example (the measurement value changes depending on a time length from a set of a measurement reagent to a measurement).

Further, by suppressing the non-specific agglutination for the antibody, the difference in measurement values between reagent lots can be detected. Accordingly, when a measurement is performed with a new reagent lot, the need for a correction therefor at each changing time can be optimally decreased.

Further, with the immunoreaction measurement method according to the present invention, the use of a surfactant such as Tween 20, octyl glucoside, sodium lauryl sulfate (SDS), sucrose monolaurate, or CHAPS, conventionally used to inhibit the non-specific autoagglutination of an antigen or an antibody can be optimally avoided. There has been a problem in that, although these surfactants are used to inhibit the non-specific autoagglutination, if the large amount thereof is used, a binding capacity in an antigen-antibody reaction is conversely weakened. Therefore, the amount of the surfactant had to be strictly set. However, according to the present invention, the non-specific autoagglutination can be removed without using the surfactant, thereby allowing to eliminate adverse effects such as protein denaturation or the like caused by the surfactant.

### BRIEF DESCRIPTION OF THE DRAWINGS

FIG. 1 is a schematic diagram of isoelectric point electrophoresis, for an antibody, used to search for an isoelectric point for the antibody used in an embodiment of the present invention.
FIG. 2 is a schematic diagram of an electrophoretic pH titration analysis used to search for the isoelectric point for the antibody used in an embodiment of the present invention.
FIG. 3 is a graph illustrating a measurement result, for a scattered light intensity for human albumin, obtained by nephelometry under an acidic condition and using a phthalate buffer solution, according to an embodiment of the present invention.
FIG. 4 is a graph illustrating blank values at each pH in the nephelometry measurement under the acidic condition and using the phthalate buffer solution, according to an embodiment of the present invention.
FIG. 5 is a graph illustrating changes of blank values , caused by an autoagglutination which depends on a pH of a reaction solution containing a mouse anti-human albumin monoclonal antibody having an isoelectric point of 6.0, according to an embodiment of the present invention.
FIG. 6 is a graph illustrating a comparison for measured scattered light intensities between antibody reagent B and antibody reagent A in the reaction solution of pH 4.5.
FIG. 7 is a diagram illustrating a top, front, and right side perspective view (a) of an immunoreaction measurement optical cell 2, and a cross sectional view (b) of the immunoreaction measurement optical cell 2, according to an embodiment of the present invention.
FIG. 8 is a diagram illustrating operations of the immunoreaction measurement optical cell 2, according to an embodiment of the present invention.

### BEST MODE FOR CARRYING OUT THE INVENTION

In an embodiment of an immunoreaction measurement method, and a regent, a kit, and an optical cell for use therein in accordance with the present invention, a pH of a reaction solution in which an antigen which is a subject substance and an antibody thereagainst are mixed together is set to be acidic. Advantages for using an acidic solution as the reaction solution are to relax a zone phenomenon in a high concentration region for the subject substance, and to obtain a suppression effect on a reduction in a spectroscopic measurement value associated with the zone phenomenon. The pH of the acidic reaction solution is preferably set to a pH of about 4 to 6, or most preferably, in a range of about 4.5 to 5.0.

In order to minimize a possible denaturation of the antibody or the like, the reaction solution set to have the acidic pH is preferably prepared immediately before a measurement is performed.

In the immunoreaction measurement method according to the present invention, the relationship between a pI of the antibody against the subject substance and the pH of the reaction system formed by mixing the subject substance and the antibody is set to be pI>pH. Therefore, antibodymolecules are positively charged in the reaction system, and the electrical repulsion between the antibody molecules enables to suppress a non-specific agglutination between the antibody molecules.

In order to facilitate the electrical repulsion between the antibody molecules, the greater the difference (|pI-pH|) between the pI value of the antibody and the pH value of the solution, the better, and it is preferred that the |pI-pH| be set equal to or more than 0.5, more preferably, equal to or more than 1.0, and most preferably, equal to or more than 1.5.

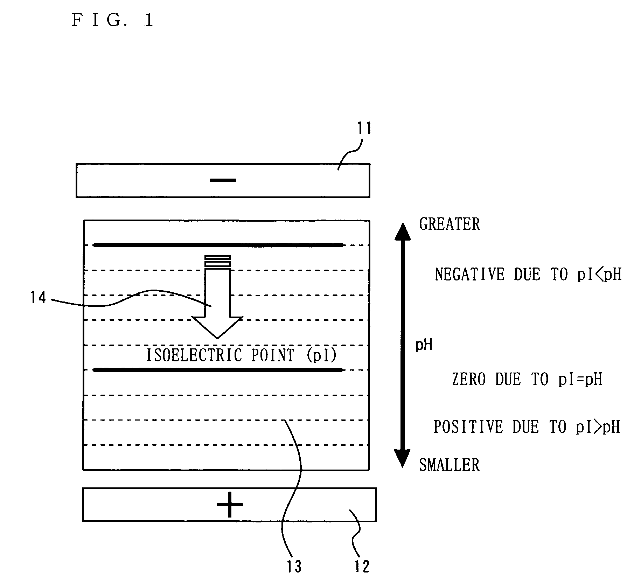

The pI of the antibody molecule can be determined by, for example, a pH titration analysis of isoelectric point electrophoresis. The analysis and selection of the antibody having a predetermined pI value by using the isoelectric point electrophoresis can be performed as follows, for example. This is described with reference to FIG. 1. By using an agarose gel for the isoelectric point electrophoresis, a pH gradient agarose gel (13) is prepared by applying a potential difference to the agarose gel immediately before a sample is put thereto. Thereafter, the sample is promptly put in the gel, and let migrate for about one hour. If the sample is negatively charged, it starts to migrate (14) toward a positive pole (12), and if the sample is positively charged, it starts to migrate toward a negative pole (11). In either case, as time passes, the sample migrates on the pH gradient agarose gel, and the closer the values between the pH on the gel and the pI of the sample become, the less the electrical charge of the sample becomes. In the end, the sample reaches in a non-electrically charged state, and then, the migration stops. The pH value for the gel at the stopped position is the pI value of the sample antibody.

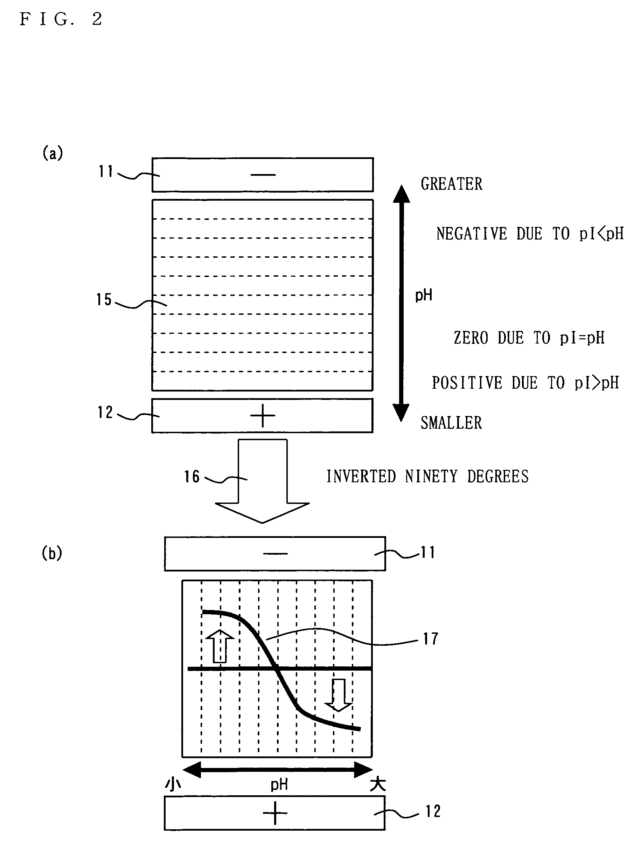

Further, the titration analysis of the sample can be performed by using an isoelectric point electrophoresis device. This is described with reference to FIGS. 2(a) and 2(b). In a similar manner to the above, a pH gradient agarose gel (15) is firstly prepared (FIG. 2(a)). Thereafter, the prepared gel is rotated ninety degrees, and set in the electrophoresis device (16) (FIG. 2(b)). Then the sample is put in the gel and let migrate for about an hour. Accordingly, the electrical charge of the sample under each pH condition can be determined (17). In other words, if the sample is on the acidic side, the sample is positively charged and therefore starts migrating toward the negative pole, and if on the alkaline side, the sample is negatively charged and therefore starts migrating toward the positive pole, simultaneously. Consequently, as illustrated by 17, a pH dependant titration curve can be obtained. The curve allows an analysis which shows: a position, of the sample, in a pH region; whether the sample is positively charged or negatively charged; and a magnitude for which the sample is charged.

A reaction solution system used in the immunoreaction measurement according to the present invention preferably contains an organic acid or an organic acid salt. The organic acid or the organic acid salt is preferably polyvalent carboxylic acid or polyvalent carboxylate salt. The polyvalent carboxylic acid refers to the organic acid having a plurality of carbonyl groups, and in the present invention, in particular, is preferred to be either: tricarboxylic acid or tricarboxylate salt; or dicarboxylic acid or dicarboxylate salt. The concentration of the tricarboxylic acid or tricarboxylate salt, or, the dicarboxylic acid or dicarboxylate salt can be arbitrary. However, in order to have significant effects on relaxing the zone phenomenon and on suppressing the reduction in a measurement value, it is preferable that the concentration not exceed 0.3 M. More preferably, the concentration of the tricarboxylic acid or tricarboxylate salt, or the dicarboxylic acid or dicarboxylate salt is selected such that it does not exceed 0.2 M, and most preferably, does not exceed 0.1 M.

As an example for the tricarboxylic acid or tricarboxylate salt, citric acid, isocitric acid, aconitic acid, and salts thereof can be given. These are commercially available in a form of, for example, anhydrous citric acid,citric acid monohydrate,trisodium citrate, trisodium citrate dihydrate, tripotassium citrate monohydrate, triammonium citrate, diammonium hydrogen citrate, calcium citrate tetrahydrate, magnesium citrate nonahydrate, trilithium citrate hydrate, copper citrate (II) 2.5 hydrate, DL-trisodium isocitrate, trans-aconitic acid, or cis-aconitic acid anhydride, and these can be used alone or in combination. Particularly, it is preferred to use the citric acid, citric acid salt, aconitic acid, or aconitic acid salt, in view that they are relatively inexpensive, can be preserved at room temperature, are available in highly stable conditions, and are easy to use.

Further, as examples for the dicarboxylic acid or the dicarboxylate salt, phthalic acid, malic acid, tartaric acid, itaconic acid, fumaric acid, maleic acid, malonic acid, succinic acid, glutaric acid, adipic acid, pimelic acid, suberic acid, azelaic acid, and salts thereof can be given. These are commercially sold in a form of, for example, phthalic acid, phthalic anhydride, potassium hydrogen phthalate, potassium phthalate, disodium phthalate, ammonium phthalate, copper phthalate (II), L(-)-malic acid, D-malic acid, DL-malic acid, sodium DL-malate, sodium L(-)-malate, L(+)-tartaric acid, DL-tartaric acid, D(-)-tartaric acid, mesotartaric acid monohydrate, potassium (+)tartrate -water(2/1), potassium sodium (+)tartrate tetrahydrate, ammonium (+)tartrate, potassium hydrogen (+)tartrate, sodium hydrogen (+)tartrate monohydrate, sodium (+)tartrate dihydrate, itaconic acid, itaconic anhydride, fumaric acid, monosodium fumarate, sodium fumarate, ferrous fumarate, maleic acid, maleic anhydride, sodium maleate, disodium maleate, malonic acid, sodium malonate, disodium malonate, thallium malonate, dithallium malonate, succinic acid, ammonium succinate, disodium succinate, glutaric acid, adipic acid, ammonium adipate, diammonium adipate, dipotassium adipate, pimelic acid, suberic acid, and azelaic acid, and these can be used alone or in combination.

In the present invention, the pH of the reaction solution or the reaction system containing the organic acid or the organic acid salt is set to be acidic, and preferably, is set in a pH range of about 4 to 6. Most preferably, the pH of the reaction system is set to a pH in a neighborhood of about 4.5 to 5.0. By setting the pH as described, the effects, i.e. , the relaxation of the zone phenomenon or the suppression for reduction in the measurement value associated with the zone phenomenon, can be obtained.

"An antibody against a subject substance" used in the immunoreaction measurement method, and in a reagent, a kit, and an optical cell for use therein in the present invention refers to an antibody which specifically binds to a subject substance based on an antigen-antibody reaction principle. Such an antibody can be, for example, a monoclonal antibody, a polyclonal antibody, a labeled antibody, or the like. The monoclonal antibody is generated by a hybridoma cell strain. The hybridoma cell strain is established by multiplying a single cell isolated from a fused cell population which has both an ability to produce an antibody and a strong ability to proliferate and is obtained by cell-fusion of a B cell producing an antibody and a bone marrow tumor cell (a myeloma cell). The polyclonal antibody can be obtained by administering an antigen into an animal thereby allowing a large quantity of antibodies which bind to the antigen to appear in the blood, by collecting all or a part of the blood, and by purifying the collected blood. In the homogenous immunoreaction measurement system in which the amount of a molecular size variation associated with the antigen-antibody reaction (complex formation) is used as an indicator for measuring the amount of a subject substance, an antibody which easily forms an agglutination complex is preferably used. Because a polyclonal antibody is an aggregate of antibodies recognizing various epitopes thereby easily forming an agglutination complex, it is preferred. The monoclonal antibody recognizes only a single epitope and forms a complex based on an 1:1 reaction, and therefore, compared to the case of the polyclonal antibody, the agglutination complex is difficult to be formed. However, in a case where, for example, the subject substance is a polyvalent antigen such as a C-reactive protein, which is a pentamer of a protein monomer, the monoclonal antibody may well be used. Also, by mixing at least two or more types of monoclonal antibodies, each recognizing a different epitope, together, a type of polyclonal antibody can be artificially designed.

Also, by labeling an antibody with a marker substance, such as a metal particle or a latex particle, the formation of an agglutination complex may be further facilitated. For example, a gold colloid labeled antibody can be produced as follows. 200 ml of gold chloride solution (manufactured by Wako Pure Chemical Industries), which is prepared such that absorbance at 290 nm is 0.86, is put into a 500 ml conical flask, and, while the gold chloride solution is being boiled, 4 ml of 1 % sodium citrate (manufactured by Wako Pure Chemical Industries) solution is swiftly added thereto. The reaction solution changes its color from blue to wine red, and is left untouched for fifteen minutes after the change is observed. The obtained gold colloid solution having been naturally cooled to the room temperature is prepared such that a pH thereof is 8.9, and an antibody and a bovine serum albumin are sequentially added thereto for labeling the antibody. After the labeling, a centrifugal separation is performed to remove an unlabeled antibody and the bovine serum albumin. As such, the gold colloid labeled antibody is produced.

The antibody used in the immunoreaction measurement method, and in the regent, the kit, and the optical cell for use therein in the present invention is not particularly limited. As long as the antibody specifically binds to a subject substance which is an antigen, any classes of antibodies, such as IgG, IgM, IgE, IgA, or IgD, may be used. Among these antibodies, it is preferred to use the IgG antibody, because the IgG antibody reacts relatively less non-specifically, and can be relatively frequently found in a commercial market thereby being easily available (commercially sold by supplier companies such as Funakoshi and Cosmo Bio, for example, but not limited thereto). Also, no specific limitation is provided to animal species from which antibodies are obtained. However, antibodies from rabbits, goats, or mice are relatively easily available, has many examples of use, and therefore, are preferred.

As a purification method, for antibodies having a predetermined pI, used in the immunoreaction measurement method, and in the reagent, the kit, and the optical cell for use therein in the present invention, various analysis methods and purification methods can be used. For example, a method, such as an isoelectric point sedimentation method, isoelectric point electrophoresis, isoelectric point chromatography, ion exchange chromatography, or the like, using a charge difference found in a substance can be used. Particularly, the isoelectric point electrophoresis is preferred.

The purified antibody can be provided, as an antibody reagent, either in a dry state or a solution state. It is preferable to preserve the antibody in a freeze-dried state for a long preservation. Such an antibody reagent may together contain an appropriate buffer. It is preferable that, before use, the aforementioned antibody reagent be preserved at around a neutral pH for preventing it from protein denaturation or the like, and, when in use, be dissolved in a buffer solution having an acidic pH for use. As an example of a buffer solution used for setting the pH at around neutral, a later described buffer solution containing 0.05M3-(N-morpholino)propansulfonic acid, 0.15MNaCl, and 0.04 wt % NaN₃, and having a pH of 7.4 can be given, but it is not limited thereto. As an example of a buffer solution having an acidic pH, a buffer solution containing the above-described organic acid or organic acid salt can be given.

In the present invention, when the reaction system is set based on the relationship between the pI value of an antibody molecule and the pH value of a solution, it is important to consider: the effect of labeling the antibody molecule; the effect of a surrounding environment for the antibody; an isoelectric range; or the like. This is because, although a pI, i.e., an isoelectfic point, for the antibody is generally in a range between about 5.0 and 8.0 (such a distribution range for the isoelectric point for an antibody is referred to as "an isoelectric range" , in the present specification), the pI can be less than 5.0 or greater than 8.0 when labeling the antibody, or depending on the surrounding environment for the antibody. In other words, the isoelectric point is affected by an ionic strength of a buffer solution, a type of the buffer solution composition, or the like. Also, the isoelectric point is also affected whether the antibody is labeled with, for example, a gold colloid, a latex particle, a color compound, or the like. Also, in the case of using the polyclonal antibody or even the monoclonal antibody, each of the individual antibody molecule has a slightly different isoelectric point from each other, and therefore, in such a solution, the isoelectric point of the dissolved antibody molecule usually exists in an "isoelectric range" fashion as described above.

The subject substance used in the immunoreaction measurement method, and in the reagent, the kit, and the optical cell for use therein in the present invention is not specifically limited, and may be a substance generally being measurable by using an antigen-antibody reaction. Proteins, nucleic acids, lipids, bacteria, viruses, haptens, and the like are examples of such substances. Particularly, proteins are preferred for being a major substance to be measured in a clinical examination. As such a protein, for example, hormones such as LH (luteinizing hormone), FSH (follicle stimulating hormone), and hCG (human chorionic gonadotropin) can be given, or, various immunoglobulin classes or subclasses, complement components, various markers for infectious diseases, C-reactive proteins, albumins, rheumatoid factors, blood-type antigens, and the like can also be given.

In the immunoreaction measurement in accordance with the present invention, in order to measure the amount of an antigen-antibody complex, it is preferable to use a method in which an optical parameter attributed to changes, in the amount and the size of a molecule, caused by the complex formation is measured. Particularly, the optical parameter is preferred to be an optical scattered light intensity or absorbance.

The kit for measuring a subject substance in a specimen, under an acidic pH condition provided in an aspect of the present invention and based on an antigen-antibody reaction principle, includes a buffer solution and a reagent containing an antibody against the subject substance, and the aforementioned reagent is prepared such that the pI of the above-described antibody with respect to the above-described acidic pH is pI > pH. In configuring the kit of the present invention, the aforementioned antibody reagent may be provided in a state where the reagent is contained in a solution, or in a dry state. In view of stability in preservation, it is preferable that the aforementioned antibody reagent be provided in a freeze-dried state.

An optical cell for measuring a subject substance in a liquid specimen under an acidic pH condition provided in an aspect of the present invention, based on the antigen-antibody reaction principle, has an aperture portion used for accepting the liquid specimen, and holds, in the optical cell, an antibody against the subject substance so as to be dissolvable in the liquid specimen. Here, the cell is configured such that a pH of a reaction system formed by mixing the liquid specimen and the antibody is acidic, and the relationship between the pH of the reaction system and a pI of the antibody is pI > pH. A buffer may further be held in the optical cell.

In the optical cell of the present invention, it is preferable that the above-described buffer be the aforementioned organic acid or the organic acid salt. In view of solubility, the organic acid or the organic acid salt and the antibody against the aforementioned subject substance held in the above-described cell so as to be movable are preferred to be provided in a freeze-dried state. Further, the aforementioned organic acid or the organic acid salt and the antibody against the above-described subject substance may be mixed together and freeze-dried, or may be individually freeze-dried.

In the optical cell of the present invention, it is preferable that a connection aperture portion for connecting to a plunger be further provided to take in a liquid specimen from the aperture portion by using the plunger. Accordingly, the accepting the liquid specimen into the optical cell becomes easy.

As a material for the optical cell of the present invention, a material having substantially flat surfaces and being optically transparent in a visible light region is preferred, and examples of such are a silica glass, a polystyrene, a polypropylene, or the like. Particularly, the polystyrene is preferred. Further, a material allowing a transmitted light measurement and a scattered light measurement is preferred.

Based on the above description and the below-described exemplary example, those skilled in the art can configure, without requiring an excessive experiment, a reaction system for the immunoreaction measurement according to the present invention in which a pI of an antibody and a pH of a reaction solution are adjusted.

Hereinafter, the present invention is described more specifically using examples, but the present invention is not limited to these examples.

### (example)

(comparative example) an analysis of human albumin under an acidic condition by using nephelometry

A buffer solution and the like described below were prepared using pure water filtered with MILLI-Q SP TOC (manufactured by Millipore). Also, a reagent, such as a salt and a buffer which are not particularly described in the following, used herein was a special grade reagent manufactured by Wako Pure Chemical Industries. However, for a polyethylene glycol 6,000, a first class reagent was used.

Firstly, an antibody reagent was prepared. A rabbit anti-human albumin polyclonal antibody was purified, by using protein A (manufactured by Amersham-Pharmacia) column chromatography, from an antiserum collected from rabbits immunized with human albumin. The purification was performed as follows: protein A was equilibrated in a column by using a binding buffer solution containing 1. 5 M glycine, and 3.0 M NaCl, and having a pH of 8.9, and thereafter, the antiserum was applied to the column allowing the antibody in the antiserum to be specifically absorbed to the protein A, thereby separating the antibody from other components in the antiserum. After the separation, an elution buffer solution having 0.1 M citric acid and a pH of 4.0 was passed through the column to elute the antibody from the protein A for collection. The eluted and collected antibody was placed to a dialysis tube with a molecular weight cut-off of ten thousand, and a dialysis was performed with a buffer solution (5L × 2 times) containing 0.05 M 3-(N-morpholino)propansulfonic acid (manufactured by Dojin, hereinafter abbreviated as a MOPS), 0.15 M NaCl, and 0.04 wt % NaN₃, and having a pH of 7.4 to substitute components in the buffer solution. Insoluble matters in the antibody solution after the dialysis were removed by a centrifugal separation at 10,000 g. The antibody concentration was measured based on a measurement of absorbance at 280 nm, and lastly, was adjusted to 3.0 mg/ml with the buffer solution used in the dialysis, and the resultant solution was regarded as an antibody reagent (antibody reagent A).

Subsequently, the buffer solutions containing polyvalent carboxylic acid or polyvalent carboxylate salt were prepared as follows. Phthalic acid was used for the polyvalent carboxylic acid. Potassium hydrogen phthalate, which was measured such that the final concentration is 0.05 M, and polyethylene glycol 6000, which was measured such that the final concentration is 4 wt %, were dissolved in pure water having about 90% of a target preparation volume. By using NaOH, the pHs of the buffer solutions were adjusted to be 4.0, 4.5, 5.0, 5.5, and 6.0.

For an immunoreaction measurement, a spectrofluorometer (manufactured by Shimadzu Corporation, model number: RF-5300PC) was used. A high-temperature cell holder (manufactured by Shimadzu Corporation, model number: 06-15440) was placed in a sample chamber of the spectrofluorometer, and was connected to a constant-temperature bath (manufactured by TAITEC, trade name: COOLNIT BATH EL-15) to circulate water kept at a temperature of twenty-five degrees Celsius for maintaining a constant temperature at a time of measurement. Conditions for the measurement with the spectrophotometer were set such that wavelengths for both excitation light and fluorescent light were 670 nm, band widths for both the fluorescence side and the excitation side were 3 nm, and sensitivity was high.

The measurement was performed as follows. 2.87 ml of the buffer solution containing the above-described polyvalent carboxylic acid was mixed with 0.1 ml of the antibody reagent A by stirring, and then, 0.03 ml of human albumin solution of a predetermined concentration was added thereto, followed by mixing by stirring, to prepare a measurement reaction solution. The reaction solution was transferred to a quartz cell for a fluorescence analysis. The cell was placed in the spectrofluorometer, and a T-type thermocouple (obtained from RS Components, model number: 219-4696) for a temperature measurement was immersed in the cell. A spectroscopic measurement was performed with a time-course measurement which starts two minutes after mixing the human albumin, and continues for 300 seconds during which a measurement was performed at an interval of 0.04 seconds. Temperature during the measurement was monitored with a digital multithermometer (manufactured by Advantest, model number: TR2114) connected to the T-type thermocouple. The obtained values measured during the 200 to 300 seconds were averaged, and the obtained average value was regarded as a measurement value for the reaction solution. This procedure was performed at various human albumin concentrations.

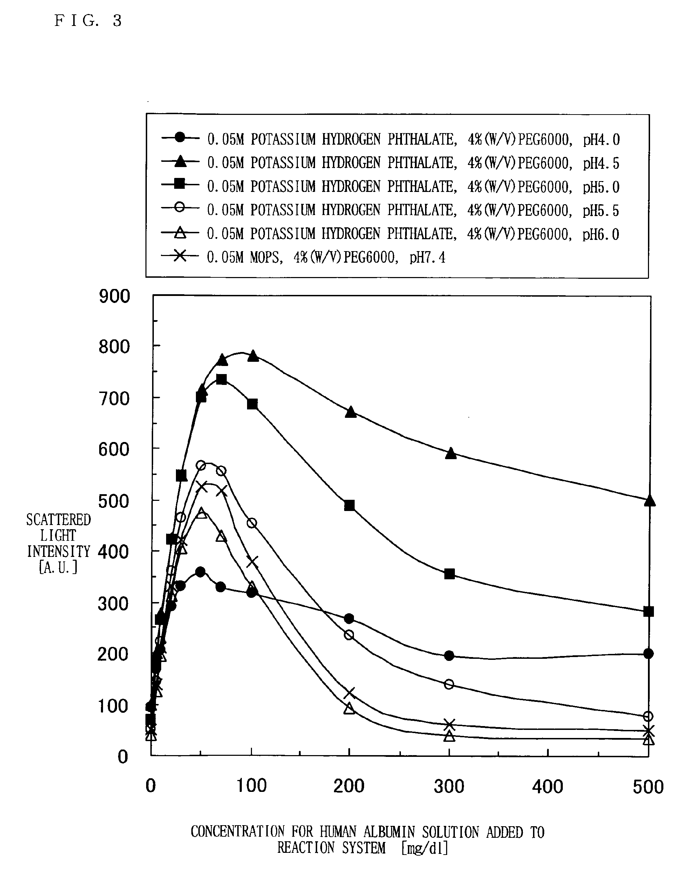

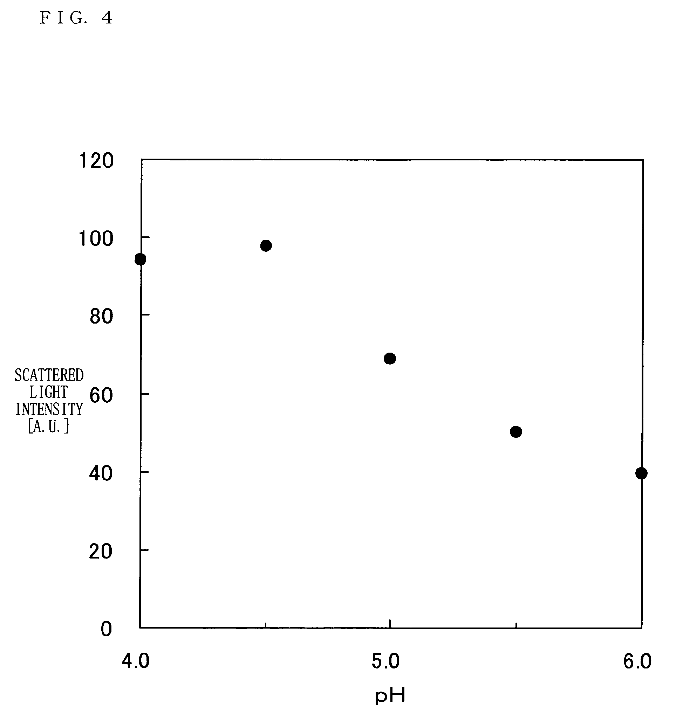

The results are illustrated in FIGS. 3 and 4. FIG. 3 shows plots having the scattered light intensity on the vertical axis and the human albumin concentration on the horizontal axis. It is observed that, depending on pHs (pHs of 4.0, 4.5, 5.0, 5.5, and 6.0) of the reaction solution, the zone phenomenon is relaxed and reduction in the measurement values is suppressed. The suppression effect on the reduction in the measurement values is most prominent especially at the pH of 4.5.

FIG. 4 shows plots having the pH of the reaction system on the horizontal axis and the scattered light intensity (blank values) when the human albumin concentration is zero on the vertical axis. It is observed that the blank value is high at a pH equal to or lower than 5.0, particularly, at a pH equal to or lower than 4.5.

The reason for the above was considered to be caused by the rough isoelectric field for the used rabbit anti-human albumin polyclonal antibody being around a neighborhood of 4.5 to 7.4. Particularly, under the acidic condition where the pH is equal to or lower than 5.0, an effect on protein denaturation can be conceived. It was considered that, in addition to the effect of the denaturation, an electrical repulsive force among the antibody molecules having an isoelectric point in the neighborhood of the pH was weakened causing stronger non-specific absorption among the antibody molecules to occur, thereby affecting the scattered light intensity which, in effect, makes the blank values particularly high.

### (example 1) checking for the effect which the relationship between a pH and a pI of an antibody has on a blank value

The effect which the relationship between a pH and a pI of an antibody has on a blank value was checked for by using a monoclonal antibody of a constant property. Anti-human albumin mouse monoclonal antibody FU-302 (manufactured by Nippon Biotest Laboratories Inc.) was used for the antibody. The pI of the antibody used was at a pH of about 6, based on a determination from isoelectric point electrophoresis. An antibody concentration was measured by an absorbance measurement at 280 nm, and made to be 3.0 mg/ml by diluting with a PBS buffer solution (8 g/l NaCl, 0.2 g/l KCl, 1.15 g/l Na₂HPO₄·12H₂O, 0.2 g/l KH₂PO₄, pH 7.4) containing 0.04 wt % NaN₃.

Buffer solutions including 0.025 M 2-(N-morpholino) ethanesulfonic acid (manufactured by Dojin, hereinafter abbreviated as a MES), 0.025 M MOPS, and 4 wt % polyethylene glycol 6000, and having pHs of 4.0, 4.5, 5.0, 5.5, 6.0, 6.5, 7.0, and 7.5 were used.

For a measurement device, a self-made device as the following was used. As a light source, a semiconductor laser pointer (manufactured by Kikoh Giken Co., Ltd.), model number: MLXS-D-12-680-35) having a wavelength of 680 nm modulated at 270 Hz and an output power of about 15 mW was used. As a detection unit, a visible and infrared light precision measurement silicon photodiode (manufactured by Hamamatsu Photonics K.K., model number: S2387-66R) was used. A sampling cell was made by attaching optical glass plates having a thickness of 0.1 cm together so as to have a shape of square prism whose volume was 200µl, for use.

The cell was placed 0.5 cm away from the light source such that one side of the cell is perpendicular to the light source, and the detection unit was placed 5.5 cm away from the cell such that the unit forms at an angle of ninety degrees with respect to the direction of light from the light source. A light shielding tube was provided between the detection unit and the cell so as to prevent stray light from entering into the detection unit. An electrical current signal depending on the amount of light detected by the detection unit was amplified, via a current-voltage conversion circuit (10⁶V/A) and an amplifier circuit of an operational amplifier, to a 100-fold voltage signal. Thereafter, the voltage signal was passed through a lock-in amplifier (manufactured by NF Kairo Sekkei Block Corporation, model number: 5610B) for performing a phase-sensitive detection, thereby allowing, based on a GPIB control, to be inputted into a computer.

The measurement of a blank value for each buffer solution was performed as follows. A mixture ratio for the reaction solution was 187µl of the buffer solution and 7µl of the antibody solution. The final concentration of the antibody in the reaction solution was about 0.11 mg/ml.

The buffer solution and the antibody solution, both having the above-described volumes, were added into the cell, and mixed by stirring. A measurement for scattered light was started 10 seconds before the addition of the antibody solution, and continued for 300 seconds during which a measurement was performed at an interval of 0.5 seconds. The measurement values were obtained as voltage values. An average value was calculated for the measurement values obtained over the 200 to 300 seconds, and the obtained average value was regarded as a blank value for each buffer solution. The measurement was performed at room temperature (about twenty-five degrees Celsius).

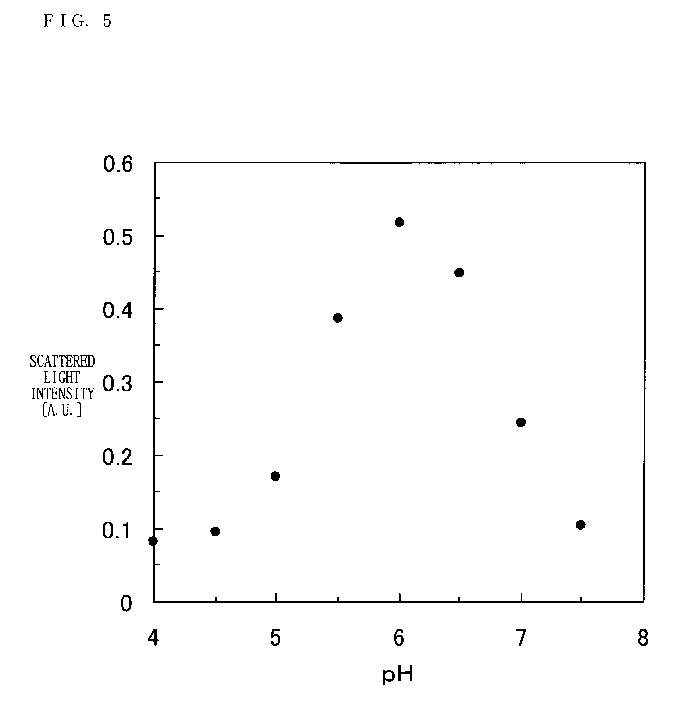

The result is shown in FIG. 5. In FIG. 5, the vertical axis represents the scattered light intensity, and the horizontal axis represents the pH. As shown, the blank value was high in the neighborhood of pH 5.5 to 6.5, i.e., in the neighborhood of the isoelectric point (about 6.0) of the used antibody, but a suppression on the blank value was already observed at a pH 0.5 lower than the isoelectric point, i.e., the pH of 5.5, and a remarkable (about one third) suppression on the blank value was observed at a pH lower than the isoelectric point of the antibody by an amount equal to or more than 1.0, i.e., the pH equal to or lower than 5.0, that is pI - pH ≥ 1.0. Accordingly, it was found that a reduction effect on the blank value was remarkably strong in a case of a condition in which a pH was lower than the isoelectric point by an amount equal to or more than about 1.0. Particularly, the effect was strong at a pH equal to or lower than 4.5, that is pI - pH ≥ 1.5, showing nearly a maximum effect, and the blank value was suppressed to about one fifth.

### (example 2) making of a human albumin measurement reaction reagent kit

In consideration of the above result, a human albumin measurement reaction reagent kit to react at a pH of 4.5 was made.

In view of the above result, in an immunoreaction measurement method such as nephelometry, in order to further suppress a blank value while maintaining the effects, i.e., relaxation in the zone phenomenon and prevention in the reduction of measurement values, it was considered that an antibody having its isoelectric field in a pH range as further away as possible with respect to a pH of an acidic buffer solution may be used as a test reagent. In view of the result shown in FIG. 4, it was considered most desirable to select an antibody having its isoelectric field in a range at least equal to or more than 1.0 away from the pH of the reaction solution (measurement solution). An antibody reagent can be made by combining two or more types of monoclonal antibodies having an isoelectric point at a pH equal to or more than 5.5 that is at least equal to or more than 1.0 away from a pH of the reaction solution and binding to different epitopes of human albumin. However, in the present example, a configuration method for an antibody reagent using a polyclonal antibody was described.

Firstly, the antibody reagent was prepared. A rabbit anti-human albumin polyclonal antibody was purified, by using protein A column chromatography, from an antiserum same as used in the comparative example. In the purification, protein A was equilibrated in a column by using a binding buffer solution containing 1. 5 M glycine, and 3.0 M NaCl, and having a pH of 8.9, and thereafter, the antiserum was applied to the column allowing the antibody in the antiserum to be specifically absorbed to the protein A, thereby separating the antibody from other components in the antiserum. After the separation, an elution buffer solution having 0.1 M citric acid, and a pH of 4.0 was passed through the column to elute the antibody from the protein A for collection. The eluted and collected antibody was placed to a dialysis tube with a molecular weight cut-off of ten thousand, and a dialysis was performed with a buffer solution (5L × 2 times) containing 0.05 M 2-(N-morpholino)ethanesulfonic acid (manufactured by Dojin, hereinafter abbreviated as an MES), and 0.04 wt % NaN₃, and having a pH of 5.5 to substitute components in the buffer solution.

Insoluble matters in the antibody solution after the dialysis were removed by a centrifugal separation at 10,000 g, and then, applied to DEAE anion exchange column chromatography for collecting non-absorbing fractions. Through the operation, an antibody positively charged at a pH of 5.5, namely, an antibody whose pI is greater than 5.5, can be selectively collected. A dialysis was performed for the collected non-absorbing fractions with a buffer solution (5L × 2 times) containing 0.05 M 3-(N-morpholino)propansulfonic acid (manufactured by Dojin, hereinafter abbreviated as a MOPS), 0.15 M NaCl, and 0.04 wt % NaN₃, and having a pH of 7.4 for substituting components in the buffer solution. Insoluble matters in the antibody solution after the dialysis were removed by the centrifugal separation at 10,000 g. The resultant antibody solution was concentrated by ultrafiltration, and thereafter, the antibody concentration was measured by using an absorbance measurement at 280 nm, and was adjusted to finally be 3.0 mg/ml by using the buffer solution used in the dialysis. The resultant antibody solution was regarded as an antibody reagent (antibody reagent B). The antibody reagent B only contains an antibody having its isoelectric field in a range of pI values 5.5 to 7.4.

The antibody concentration adjusted as in the above is not particularly limited thereto. The antibody solution having been made can be preserved at room temperature, but, from the point of view of preventing the antibody denaturation, preserving the antibody at a lower than room temperature is preferable, and preserving the antibody at four degrees Celsius is more preferable.

Next, a buffer solution containing polyvalent carboxylic acid or polyvalent carboxylate salt was prepared as follows. Phthalic acid was used for the polyvalent carboxylic acid. Potassium hydrogen phthalate, which was measured such that the final concentration is 0. 05 M, and polyethylene glycol 6000, which was measured such that the final concentration is 4 wt %, were dissolved in pure water having about 90% of a target preparation volume. A pH of the buffer solution was adjusted to be 4.5 by using NaOH.

Through combining the buffer solution configured as above and containing the polyvalent carboxylic acid or the polyvalent carboxylate salt with the prepared antibody reagent prepared in the above, a human albumin measurement reaction reagent kit can be configured.

In an event of using the human albumin measurement reaction reagent kit configured as above, a sample (specimen) containing human albumin, an antibody reagent, and a buffer solution containing polyvalent carboxylic acid or polyvalent carboxylate salt are mixed together and then used for forming a reaction system.

The aforementioned mixing may be performed by an arbitrary method. The mixing ratio can be determined in accordance with a measurement range for a required human albumin concentration. By measuring a variable (for example, the amount of antigen-antibody complex) caused by an immunoreaction caused between the human albumin generated in the reaction system formed by mixing and the antibody, an antigen concentration in the specimen can be determined.

Note that the antibody may be immobilized on a fine particulate carrier such as a latex, a gold colloid, or a magnetic particle. Alternatively, the antibody may be labeled with an enzyme, a dye, a fluorescent substance, a luminescent substance, or the like.

Note that, in the preparation for the above-described kit, NaOH was used for adjusting the pH, but hydroxide such as KOH, LiOH, NH₄OH, Ca(OH)₂, Mg(OH)₂, or the like may well be used.

Further, in the example, the potassium hydrogen phthalate was used for adjusting the buffer solution containing the polyvalent carboxylic acid or the polyvalent carboxylate salt. However, one of the other polyvalent carboxylic acids or polyvalent carboxylate salts, for example, anhydrous citric acid, citric acid monohydrate, trisodium citrate, trisodium citrate dihydrate, tripotassium citrate monohydrate, triammonium citrate, diammonium hydrogen citrate, calcium citrate tetrahydrate, magnesium citrate nonahydrate, trilithium citrate hydrate, copper citrate (II) 2.5 hydrate, DL-trisodium isocitrate, trans-aconitic acid, cis-aconitic acid anhydride, phthalic acid, phthalic anhydride, potassium phthalate, disodium phthalate, ammonium phthalate, copper phthalate (II), L(-)-malic acid, D-malic acid, DL-malic acid, sodium DL-malate, sodium L(-)-malate, L(+)-tartaric acid, DL-tartaric acid, D(-)-tartaric acid, mesotartaric acid monohydrate, potassium(+)tartrate-water(2/1), potassium sodium (+) tartrate tetrahydrate, ammonium (+) tartrate, potassium hydrogen (+)tartrate, sodium hydrogen (+)tartrate monohydrate, sodium (+)tartrate dihydrate, itaconic acid, itaconic anhydride, fumaric acid, monosodium fumarate, sodium fumarate, ferrous fumarate, maleic acid, maleic anhydride, sodium maleate, disodium maleate, malonic acid, sodium malonate, disodium malonate, thallium malonate, dithallium malonate, succinic acid, ammoniumsuccinate, disodium succinate, glutaricacid, adipicacid, ammonium adipate, diammonium adipate, dipotassium adipate, pimelic acid, suberic acid, azelaic acid, or the like may be used. Further, these can be combined for use. In performing the pH adjustment in such a case, if a pH at the time of dissolving in pure water is more on the alkaline side than a targeted pH for the adjustment, an HCl or the like may be used, and, if on the acid side, the above-described hydroxides or the like may be used. Further, the mixing ratio for the polyvalent carboxylic acid or the polyvalent carboxylate salt that is exemplary described in the above may be modified and is used.

Further, the buffer components and the pH of the antibody solution are not limited to the aforementioned composition and the pH. For example, in a case where a reagent of single liquid type, namely, a test solution, is configured, in order for the antibody solution to contain the polyvalent carboxylic acid or the polyvalent carboxylate salt and to have an acidic pH for a reaction system, the dialysis may be performed with a buffer solution which contains the targeted polyvalent carboxylic acid or polyvalent carboxylate salt, and is adjusted to have the targeted acidic pH.

Also, in the present example, a configuration method for a reagent when an anti-human albumin polyclonal antibody was used is described, but the reagent may be configured by mixing two or more types of monoclonal antibodies each binding different parts of human albumin. In such a case, the pI of the antibody to be used is to be greater than the pH of the buffer solution to be used, preferably by an amount equal to or more than 0.5, more preferably by an amount equal to or more than 1.0, or most preferably by an amount equal to or more than 1. 5. In the case of the present example, it is more preferable that the pI be equal to or greater than 5.5, and it is most preferable if the pI is equal to or greater than 6.0.

### (example 3) reduction effect, on blank values, provided by human albumin measurement reaction reagent kit

By using: the antibody reagent B which only contains an antibody having an isoelectric field in a range of pI values 5.5 to 7.4 and is prepared in example 2; and the antibody reagent A which only contains an antibody having an isoelectric field in a range of pI values 4.5 to 7.4 and is prepared in the comparative example, a spectroscopic measurement was performed in a reaction solution having a pH of 4.5 by using the procedure same as in the comparative example. As for the antibody reagent A and the antibody reagent B, components of the buffer solution at the time of measurement were identical to each other in that they contain polyvalent carboxylic acid or polyvalent carboxylate salt in the reaction solution.

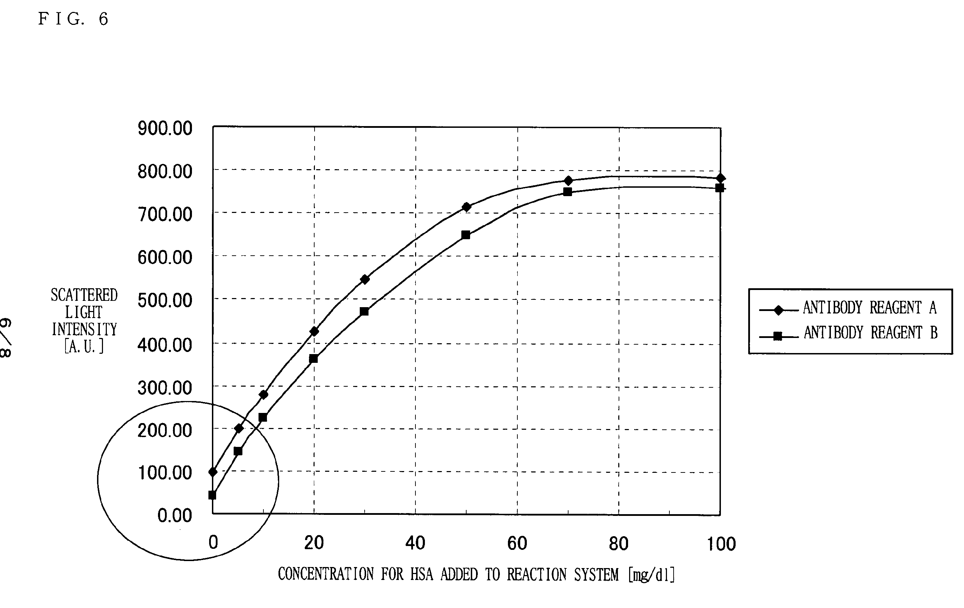

The result is shown in FIG. 6. As indicated, it is found that the scattered light intensity (i.e., blank value) for the antibody reagent B at zero concentration for human albumin added to the reaction system is remarkably reduced compared to the blank value for the antibody reagent A used in the comparative example.

As such, by selecting and using an antibody having an appropriate isoelectric field for a pH of the used buffer solution, an increase of the blank value in the immunoreaction measurement method can be suppressed, whereby the validity of the human albumin measurement reaction reagent kit according to an example of the present invention was confirmed.

### (example 4) structure of an immunoreaction measurement optical cell

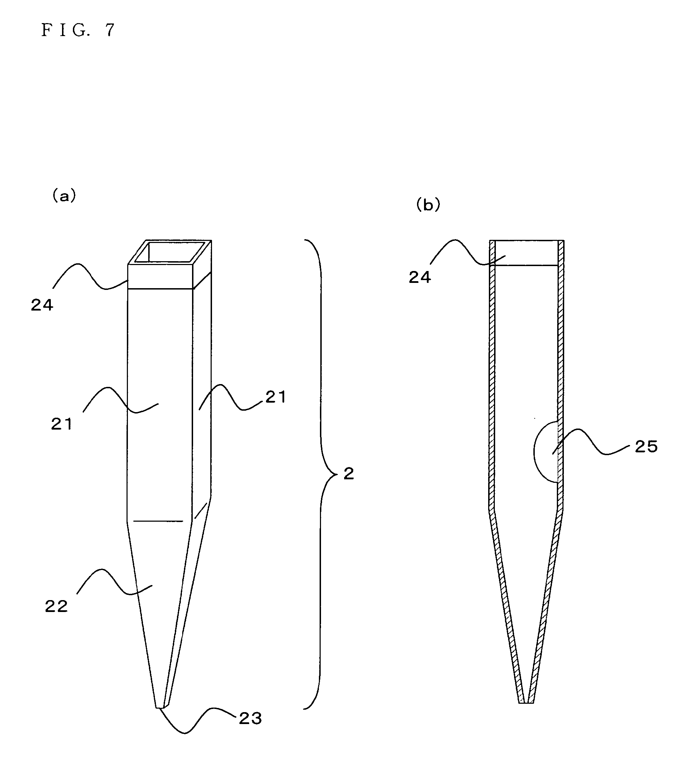

Hereinafter, an immunoreaction measurement optical cell of the present invention is described in detail with reference to the diagrams. FIG. 7(a) is a top, front, and right side perspective view illustrating a structure of an immunoreaction measurement optical cell 2 of the present invention, and FIG. 7(b) is a cross sectional view thereof.

The immunoreaction measurement optical cell 2 in the present experiment includes an optical measurement window 21, a sample introduction path 22, an aperture portion 23, and a plunger connection portion 24. In the present example, the three-dimensional shape of a part for which the optical measurement window 21 of the optical cell 2 was provided was a square prism, and each of the four surfaces thereof was substantially flat, and constructed from an optically transparent material in a visible light region. It was configured such that an optical path length was 1 cm, about 1 ml of a sample intake was possible, and an optical measurement could be performed. In the present example, a polystyrene was used for the cell material.

A reagent 25 was held, by freeze-drying, on the inner wall between the optical measurement window 21 of the immunoreaction measurement optical cell 2 and the sample introduction path 22 so as to be movable. In the present example, the reagent 25 was prepared such that, when introducing 1 ml of liquid sample, the final concentrations of ingredients were: 1 mg/ml human albumin antibody; 0.05 M phthalic acid; and 4 wt % polyethylene glycol 6000, and a pH thereof was 5.0.

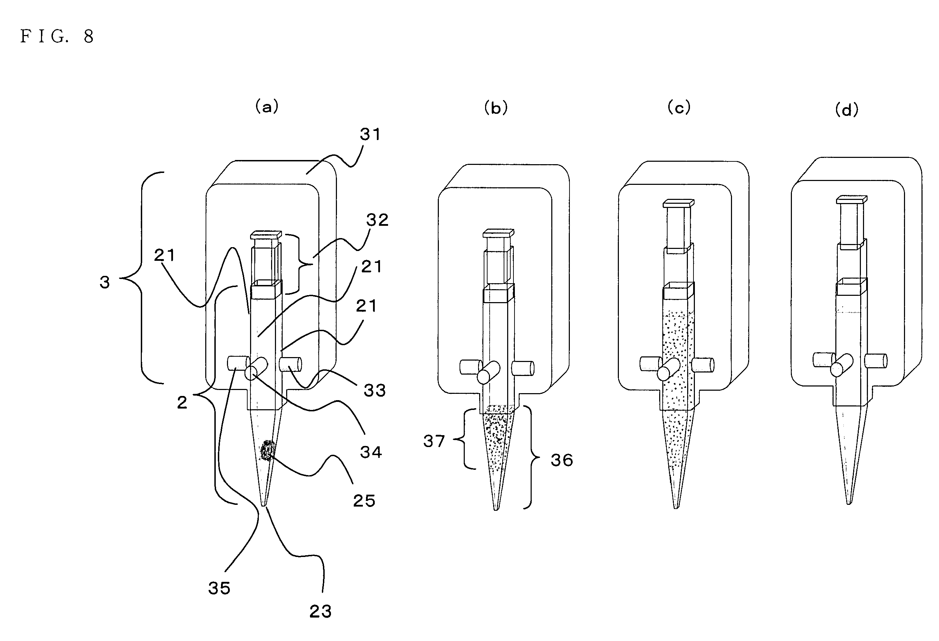

Next, a configuration outline of the immunoreaction measurement optical cell 2 configured as above is described with reference to FIGS. 8(a) to 8(d).

FIG. 8(a) schematically illustrates a general configuration of an analysis apparatus 3 having the immunoreaction measurement optical cell 2 installed therewith. In the present example, the analysis apparatus 3 includes, in a cabinet 31, a detection portion including: a plunger 32 connected to the immunoreaction measurement optical cell 2; a light source 35 vertically arranged on one side of the optical measurement window 21 of the immunoreaction measurement optical cell 2; a transmitted light detector 33 vertically arranged, on another side of the optical measurement window 21, on a transmitted light path of the light source 35; and a scattered light detector 34 vertically arranged, on still another side of the optical measurement window 21, at the same height as the transmitted light path of the light source 35.

As illustrated in FIGS. 8(b) to 8(d), by lifting the plunger 32 upward, a liquid sample 36 is flowed in from the aperture portion 23. Through the inflow of the liquid sample 36, as illustrated in FIG. 8(b), the reagent 25, which is held, by freeze-drying, on the inner wall of the optical measurement window 21 of the immunoreaction measurement optical cell 2 so as to be movable and is composed of a human albumin antibody, phthalic acid, and polyethylene glycol 6000, touches the liquid sample 36, causing the reagent 25 to be dispersed in the liquid sample 36 and to start dissolving (a reagent 37 dispersed in the liquid sample). As illustrated in FIG. 8(c), by further lifting the plunger 32 upward, the liquid sample 36 reaches a higher portion than the portion where the light source 35 on the optical measurement window 21 of the immunoreaction measurement optical cell 2, the transmitted light detector 33, and the scattered light detector 34 are provided.

Through the flux caused by the inflow of the liquid sample 36 from the aperture portion 23, the reagent 37, which is composed of the human albumin antibody, phthalic acid, and polyethylene glycol 6000 and is dispersed in the liquid sample, finally dissolves in the liquid sample 36, as illustrated in FIG. 8(d). Accordingly, an agglutination complex is generated by an antigen-antibody reaction caused by the human albumin and the human albumin antibody included in the liquid sample 36, whereby turbidity is generated in the liquid sample.

The degree of the turbidity depends on the concentration of the human albumin contained in the liquid sample 36 , and therefore, by measuring the degree of the turbidity, the concentration of the human albumin contained in the liquid sample 36 can be determined.

In order to measure the degree of the turbidity in the liquid sample 36, light is irradiated, from the light source 35, directing approximately vertically to one side of the optical measurement window 21. The light entered from the one side of the optical measurement window 21 is transmitted through the liquid sample 36, and then, the light traveling in a straight direction is outputted through the optical measurement window 21 opposite to the optical measurement window 21 of the side of the light source 35, and received by the transmitted light detector 33. The light scattered by the agglutination complex, caused by the antigen-antibody reaction, in the liquid sample 36 is outputted through the optical measurement window 21 vertical to the optical measurement window 21 of the side of the light source 35 , and received by the scattered light detector 34. Here, although not shown in the diagrams, the output from each of the detectors is A/D converted, inputted into a microcomputer or a PC, and data processed.

Based on at least one of the light intensities received by the transmitted light detector 33 and the scattered light detector 34, a reaction generated between the sample and the immunoreaction measurement reagent can be measured. When the light intensity from the transmitted light detector 33 is used, absorbance or turbidity is determined. In this case, transmitted light before the introduction of the liquid sample 36 is previously regarded as a reference light.

When the light intensity from the scattered light detector 34 is used, the scattered light intensity is determined. Although either of the indicators may be used for measuring the reaction between the sample and the reagent, if the degree of the turbidity caused by the reaction between the sample and the reagent is low, using the scattered light intensity allows a more sensitive measurement.

As described above, according to the immunoreaction measurement optical cell 2 in the present example, a spectroscopic measurement for an antigen-antibody reaction can be swiftly performed simultaneous to a sampling of a sample, thereby firmly allowing, especially when measuring transient changes of the reaction, an observation of the transient changes with less time-loss. Further, there is no need to transfer a liquid sample - a reagent mixed solution to an optical cell, thereby allowing an easy operation.

While the invention has been described in detail, the foregoing description is in all aspects illustrative and not restrictive. It is understood that numerous other modifications and variations can be devised without departing from the scope of the invention.

### INDUSTRIAL APPLICABILITY

An immunoreaction measurement method, and a reagent, a kit, and an optical cell for use therein according to the present invention have effects in that a zone phenomenon in an immunoreactionmeasurement for a specimen is suppressed, reduction in a measurement value is suppressed, and a non-specific agglutination between antibody molecules is suppressed, and therefore, are especially useful as the immunoreaction measurement method, the reagent, the kit, the optical cell, or the like using a homogeneous measurement system such as nephelometry, turbidimetry, slide agglutination, or the like.

## Claims

1. A method for measuring a subject substance in a specimen under an acidic pH condition, comprising:
step A of forming a reaction system by mixing the specimen and an antibody against the subject substance in the specimen; and
step B of measuring an antigen-antibody reaction in the reaction system, wherein
a pH of the reaction system is set to be acidic, and
a relationship between a pI of the antibody and the pH of the reaction system is set to be pI > pH.

2. The method according to claim 1, wherein, in the step B, an amount of an antigen-antibody complex formed between the subject substance and the antibody is measured.

3. The method according to claim 1, wherein the difference between the pI and the pH is equal to or more than about 1.0.

4. The method according to claim 1, wherein the difference between the pI and the pH is equal to or more than about 1.5.

5. The method according to claim 1, wherein the pH is in a range of about 4 to 6.

6. The method according to claim 1, wherein the pH is in a range of about 4.5 to 5.0.

7. The method according to claim 1, wherein, in the step A, the reaction system is formed by mixing the specimen, the antibody, and a buffer.

8. The method according to claim 1, wherein the reaction system contains an organic acid or an organic acid salt.

9. The method according to claim 8, wherein the organic acid or the organic acid salt is a polyvalent carboxylic acid or a polyvalent carboxylate salt.

10. The method according to claim 9, wherein the polyvalent carboxylic acid or the polyvalent carboxylate salt is either: a tricarboxylic acid or a tricarboxylate salt; or a dicarboxylic acid or a dicarboxylate salt.

11. The method according to claim 1, wherein the antibody is a monoclonal antibody, a polyclonal antibody, or a labeled antibody.

12. The method according to claim 2, wherein the step B of measuring includes a step of measuring an optical parameter attributed to the amount or the size of the complex.

13. A reagent for measuring a subject substance in a specimen under an acidic pH condition based on an antigen-antibody reaction principle, the reagent including an antibody against the subject substance, wherein a pI of the antibody with respect to the acidic pH is prepared to be pI > pH.

14. The reagent according to claim 13, wherein the difference between the pI and the pH is equal to or more than about 1.0.

15. The reagent according to claim 13, wherein the difference between the pI and the pH is equal to or more than about 1.5.

16. The reagent according to claim 13, wherein the pH is in a range of about 4 to 6.

17. The reagent according to claim 13, wherein the pH is in a range of about 4.5 to 5.0.

18. The reagent according to claim 13, wherein an organic acid or an organic acid salt is contained.

19. The reagent according to claim 18, wherein the organic acid or the organic acid salt is a polyvalent carboxylic acid or a polyvalent carboxylate salt.

20. The reagent according to claim 19, wherein the polyvalent carboxylic acid or the polyvalent carboxylate salt is either: a tricarboxylic acid or a tricarboxylate salt; or a dicarboxylic acid or a dicarboxylate salt.

21. The reagent according to claim 13, wherein the antibody is a monoclonal antibody, a polyclonal antibody, or a labeled antibody.

22. The reagent according to claim 13 provided in a freeze-dried state.

23. The reagent according to claim 13 provided as a solution.

24. A measurement kit for measuring a subject substance in a specimen under an acidic pH condition based on an antigen-antibody reaction principle, the kit including:
a buffer solution; and
a reagent including an antibody against the subject substance,
wherein
the reagent is prepared such that a pI of the antibody with respect to the acidic pH is pI > pH.

25. The kit according to claim 24, wherein the difference between the pI and the pH is equal to or more than about 1.0.

26. The kit according to claim 24, wherein the difference between the pI and the pH is equal to or more than about 1.5.

27. The kit according to claim 24, wherein a pH of the buffer solution is in a range of about 4 to 6.

28. The kit according to claim 24, wherein the pH of the buffer solution is in a range of about 4.5 to 5.0.

29. The kit according to claim 24, wherein the buffer solution contains an organic acid or an organic acid salt.

30. The kit according to claim 29, wherein the organic acid or the organic acid salt is a polyvalent carboxylic acid or a polyvalent carboxylate salt.

31. The kit according to claim 30, wherein the polyvalent carboxylic acid or the polyvalent carboxylate salt is either: a tricarboxylic acid or a tricarboxylate salt; or a dicarboxylic acid or a dicarboxylate salt.

32. The kit according to claim 24, wherein the antibody is a monoclonal antibody, a polyclonal antibody, or a labeled antibody.

33. The kit according to claim 24, wherein the antibody is a mixture of at least equal to or more than two types of the monoclonal antibodies each recognizing a different epitope.

34. The kit according to claim 24, wherein the reagent is provided in a freeze-dried state.

35. The kit according to claim 24, wherein the buffer solution and the reagent are mixed together, in order to measure the subject substance, immediately before the use and the mixture is used as a test solution.

36. An immunoreaction measurement optical cell for measuring a subject substance in a liquid specimen under an acidic pH condition based on an antigen-antibody reaction principle, the optical cell having
an aperture portion for accepting the liquid specimen,
wherein
an antibody against the subject substance is held in the optical cell so as to be dissolvable in the liquid specimen, and
a pH of a reaction system formed by mixing the liquid specimen and the antibody against the subject substance is acidic, and a relationship between the pH of the reaction system and the pI of the antibody is pI > pH.

37. The optical cell according to claim 36 further comprising a buffer in the optical cell.
